## Europäisches Patentamt
## European Patent Office
## Office européen des brevets

(19)

(11) Veröffentlichungsnummer: **0 234 019**
**B1**

(12) # EUROPÄISCHE PATENTSCHRIFT

(45) Veröffentlichungstag der Patentschrift:
07.03.90

(51) Int. Cl.⁴: **A61K 35/78**

(21) Anmeldenummer: **86117175.9**

(22) Anmeldetag: **10.12.86**

(54) **Verwendung von wässrigen Auszügen von Epilobium angustifolium L. zu einer neuen medizinischen Indikation.**

(30) Priorität: **19.02.86 DE 3605250**

(43) Veröffentlichungstag der Anmeldung:
**02.09.87 Patentblatt 87/36**

(45) Bekanntmachung des Hinweises auf die Patenterteilung:
**07.03.90 Patentblatt 90/10**

(84) Benannte Vertragsstaaten:
**AT BE CH DE FR GB IT LI LU NL SE**

(56) Entgegenhaltungen:
**CHEMICAL ABSTRACTS, Band 74, Nr. 3, 18.01.71,
Seite 194, Abstract 11701h, Moscow, USSR;
PUKHALSKAYA et al.: "Isolation of a polymer from
Chamaenerion angustifolium and studies on its
antitumor activity" & ANTIBIOTIKI
(MOSCOW) 1970, 15(9), 782-785
30063602 BIOLOGICAL ABSTRACTS/RRM, Inst.
Biochem., Acad. Sci. Lith. SSR, Vilnius, USSR;
VALAVICHYUS et al.: "Antitumor activity of medicinal
drugs 5. rose bay", & LIETUVOS TSR MOKSLU
AKADEMIJOS DARBAI SERIJA C BIOLOGIJOS
MOKSLAI, USSR, 1985 0(3), 100-102SKOGIND. 34,
NO. 12: 299-300, 272 (DEC. 1980) 000**

(73) Patentinhaber: **Apotheker Popp oHG,
Peterstrasse 33-39, D-8500 Nürnberg 30(DE)**

(72) Erfinder: **Hiermann, Alois, Dr. Dr., Lendkai 31,
A-8020 Graz(AT)**

(74) Vertreter: **Patentanwälte Czowalla . Matschkur +
Partner,
Dr.-Kurt-Schumacher-Strasse 23 Postfach 9109,
D-8500 Nürnberg 11(DE)**

## Beschreibung

Die Erfindung richtet auf ein Mittel zur Behandlung entzündlicher Zustände, und zwar in Form wäßriger Auszüge von Epilobium angustifolium L. Die Erfindung bezieht sich auf die erste diesbezügliche medizinische Indikation.

Epilobium ist aus der Volksheilkunde bekannt zur Anwendung als Emolliens, Solvens oder als Mucilaginosum. Seine antibakteriellen und fungiziden Wirkungen sind in diesem Rahmen gleichfalls bekannt geworden. Epilobium wurde vornehmlich als Teesurrogat verwendet, wurde aber auch zur Verwendung von Salat oder Gemüse empfohlen. Nur wenige Arbeiten haben sich mit der Pharmakologie und Phytochemie von Epilobium befaßt. Dieses kommt allerdings nur als Blattdroge in Betracht. Die Blüte kann in diesem Zusammenhang außer Betracht bleiben.

Der oxidative Stoffwechsel der ungesättigten Fettsäuren führt im Körper zur Synthese von Substanzen mit ausgeprägten biologischen Wirkungen. Die primäre Metabolisierung der Arachidonsäure durch die Cyclooxygenase führt zu den Prostaglandinen (E, F, A, B, C, D), Prostacyclin und Thromboxan. Wird die Arachidonsäure primär über die Lipoxygenase metabolisiert, entstehen die Leukotriene (z.B. LTA, LTB, LTC, LTD, LTE, LTF).

Die Prostaglandine spielen eine wesentliche Rolle bei Entstehung und Ablauf einer akuten Entzündung; sie steigern die Kapillarpermeabilität, sensibilisieren die Schmerzrezeptoren und setzen lysosomale Enzyme frei. Die normalerweise auf einem niedrigen Niveau liegende Prostaglandin-Synthese erfährt bei jeder Irritierung des Gewebes eine starke Steigerung. Das als Durchgangsstufe entstehende Endoperoxid ist biologisch wirksam, aber chemisch labil, es geht in die Prostaglandine über. Aus dem Endoperoxid können über zwei weitere Metabolisierungswege auch Prostacyclin und Thromboxan entstehen.

Auch im Zentralnervensystem spielen die Prostaglandine eine wichtige Rolle, wie z.B. bei der Fieberentstehung. Zusätzlich hemmen sie die Spontanlipolyse und die Lipolyse, die durch Katecholamine, Glucagon oder Theophyllin stimuliert wird. Darüber hinaus kommt den Prostaglandinen - vornehmlich Prostacyclin und Thromboxan - eine bedeutende Funktion bei der Steuerung des Gefäß-Blut-Organ-Systems zu.

Die Metabolisierung der Arachidonsäure über die Lipoxygenase führt zu den Leukotrienen. Die pulmonalen, kardialen und vaskulären Wirkungen der Leukotriene sind von besonderer Bedeutung. Asthma, Anaphylaxie und Entzündung werden durch Leukotriene entscheidend beeinflußt. Von Bedeutung ist, daß Leukotriene aus neutrophilen Granulozyten, Makrophagen und Mastzellen freigesetzt werden können.

Bei einer Entzündung handelt es sich um einen komplexen Vorgang, bei dem neben den Prostaglandinen offensichtlich noch andere Mediatoren wie Kinine, Histamin, Leukotriene u.a. eine bedeutende Rolle spielen. Es gibt Stoffe, die nur die Metabolisierung der Arachidonsäure durch die Cyclooxygenase und damit die Prostaglandin-Synthese verhindern. Es sind aber auch Stoffe bekannt, die sowohl die Cyclooxygenase als auch die Lipoxygenase und damit die Bildung von Prostaglandinen und Leukotrienen hemmen. Daneben gibt es Substanzen wie die Histamin-Blocker, die eine Freisetzung von Histamin blockieren können.

Die Prüfung der entzündungshemmenden Wirkung einer Substanz erfolgt dadurch, daß die Hemmung der Freisetzung der Mediatoren selbst, z.B. der Prostaglandine, bestimmt wird. Eine weitere globale Möglichkeit, die entzündungshemmende Wirkung einer Substanz zu überprüfen, bietet das Modell des Carrageenin-induzierten Rattenpfotenödems. Es handelt sich dabei um ein allgemein anerkanntes Modell für eine akute Entzündung. Bei der Entstehung der akuten Entzündung wirken allerdings eine Reihe von Mediatoren mit. Aus der Tatsache der Hemmung des Carrageenin-induzierten Rattenpfotenödems kann jedoch noch nicht geschlossen werden, welcher Mediator inhibiert wird.

Eine Hemmung der Synthese und/oder Freisetzung der Prostaglandine $E_2$, $D_2$ u.a. führt bereits zu einer Verminderung der Entzündungs-Reaktionen bzw. deren Folgen (Schmerzen). Man bedient sich deshalb verschiedener Medikamente, die über eine Hemmung der Cyclooxygenase dem Entzündungsmechanismus entgegenwirken. Die in dieser Hinsicht älteste und bekannteste Substanz ist die Acetylsalicylsäure; sie gehört wie viele Neuentwicklungen zur Gruppe der Säure-Antiphlogistika. Gewisse Stoffe hemmen zwar eine Synthese und Freisetzung bestimmter Prostaglandine, sind jedoch beim Carrageeninduzierten Rattenpfotenödem unwirksam. Andererseits sind Stoffe bekannt, die sowohl die Prostaglandin-Synthese und -Freisetzung als auch das Carrageenin-induzierte Rattenpfotenödem hemmen. Es liegt auf der Hand, daß letztere neben der Synthese und Freisetzung der Prostaglandine auch die Bildung anderer am Entzündungsgeschehen beteiligter Mediatoren hemmen.

Nebenwirkungen der Substanzen vom Typ der Säure-Antiphlogistika, die - je nach Stoff - von der Irritation der Magenschleimhaut bis zur Ulcusbildung und zum anaphylaktischen Schock reichen, begründen die Problematik ihrer Anwendung. Hieraus erwächst die Notwendigkeit der Suche nach Substanzen, die eine akute Entzündung und Prostaglandin-Synthese und -Freisetzung hemmen, jedoch weniger gravierende Nebenwirkungen aufweisen.

Es hat sich nun überraschenderweise ergeben, daß die Droge Epilobium angustifolium sich in besonderer Weise zur Behandlung entzündlicher Zustände eignet. Sie enthält Stoffe, welche die gesuchten Wirkungen entfaltet, nämlich die Hemmung einer akuten Entzündungsreaktion, nachgewiesen im Modell des Carrageenin-induzierten Rattenpfotenödems und die Hemmung der Synthese und Freisetzung von Prostaglandinen. Die Substanzen, die diese Wirkungen erzeugen, befinden sich vorzugsweise in wäßrigen Auszügen von Epilobium angustifolium. Diese kommen, wie sich ergeben hat, zur Hemmung der Metabolisierung der Arachidonsäure, insbesondere zur Hemmung der Prostaglandin-Synthese und/oder -Freisetzung bei der Behandlung von entzündlichen Zuständen in Betracht. Sie finden also Vewendung bei der Behandlung von Krankheiten, die − ganz oder teilweise − durch eine akute Entzündung und/oder die Bildung und Freisetzung von Prostaglandinen verursacht sind. Eine solche Indikation erscheint mit Sicherheit nicht nahegelegt durch die vorbekannte Nutzbarmachung des Epilobiums im Rahmen der Volksheilkunde als Sekretolytikum.

Die wäßrigen Auszüge von Epilobium angustifolium kommen bevorzugt in Betracht zur Herstellung von Arzneimitteln für die vorgegebenen Anwendungsbereiche. Von besonderer Bedeutung ist, daß diese Mittel nach peroraler Applikation eine systemische, entzündungshemmende Wirkung und eine Inhibierung der Synthese und Freisetzung von Prostaglandinen entfalten.

Die Erfindung umfaßt eine Reihe verschiedener Herstellungsverfahren für den wirksamen Extrakt, von denen im folgenden einige Beispiele angegeben werden:

Beispiel 1

5 g Herba Epilobii angustifolii plv. werden mit 250 ml (150 ml) heißem Wasser übergossen und 10 Minuten unter mehrfachem Umrühren stehen gelassen. Nach Filtration wird der Extrakt mit Natriumchlorid auf ein physiologisches Niveau gebracht, wozu ein Osmometer Verwendung findet.

Beispiel 2

5 g Herba Epilobii angustifolii plv. werden im Soxhlet-Apparat mit Aceton, Chloroform, Dichlormethan, Äthylacetat oder Äthylmethylketon erschöpfend extrahiert, anschließend an der Luft getrocknet, mit 250 ml (150 ml) heißem Wasser übergossen und 10 Minuten unter mehrfachem Umrühren stehen gelassen. Nach Filtration wird der Extrakt auf ein physiologisches Niveau gebracht.

Beispiel 3

5 g Herba Epilobii angustifolii plv. werden mit 250 ml (150 ml) heißem Wasser übergossen und 10 Minuten unter mehrfachem Umrühren stehen gelassen. Nach Filtration wird auf 100 ml eingeengt, und die so erhaltene Lösung wird in einer Vorlage von 400 ml Methanol bei 4°C unter leichtem Umrühren zugetropft. Der hierbei entstehende Niederschlag wird abzentrifugiert (4000 U/min, 4°C), in 100 ml Wasser aufgenommen und tropfenweise mit Bleiacetatlösung (10 %-ig) versetzt bis kein Niederschlag mehr entsteht. Der Niederschlag wird anschließend mit Wasser gewaschen, in 50 ml Wasser suspendiert und mit 100 ml Chloroform, in welchem 30 mg Dithizon gelöst sind, versetzt und ausgeschüttelt. Das Ausschütteln mit Dithizon wird so oft wiederholt, bis die zu verwerfende Chloroformphase grün ist. Die zuletzt verbleibende wässrige Phase wird mit Wasser auf ein Volumen von 250 ml (150 ml) aufgefüllt und auf ein physiologisches Niveau gebracht.

Beispiel 4

5 g Herba Epilobii angustifolii plv. werden mit 250 ml heißem Wasser übergossen und 10 Minuten unter mehrfachem Umrühren stehen gelassen. Nach Filtration wird die so gewonnene wässrige Lösung einer Membranfiltration mit einer Ausschlußgrenze 10.000 MG unterworfen (gesammeltes Diafiltrat 1500 ml, Filtrationsdruck 1,5 bar, $N_2$) und anschließend einer Membranfiltration mit einer Ausschlußgrenze 1000 MG unterzogen (gesammeltes Diafiltrat 1500 ml, Filtrationsdruck 4,5 bar, $N_2$). Nach Einengen des Diafiltrats auf ein Volumen von 250 ml (150 ml) wird viermal mit der gleichen Menge Äthylacetat ausgeschüttelt, und die Äthylacetatphasen werden verworfen. Die wässrige Phase wird zum Vertreiben des Äthylacetats kurz erwärmt und anschließend auf ein physiologisches Niveau gebracht.

Das bei diesen Beispielen mit 250 ml heißem Wasser behandelte Medium bzw. das auf 250 ml eingeengte Diafiltrat dient dem nachfolgenden Test der PG-Freisetzung (PG = Prostaglandine). Die Behandlung mit 150 ml heißem Wasser bzw. Einengung auf 150 ml dient dem nachfolgenden Test nach dem Rattenpfotenödem.

Der Nachweis der Beeinflussung wässriger Auszüge von Epilobim angustifolium auf die Biosynthese bzw. Freisetzung der Prostaglandine $PGE_2$, $PGI_2$ und $PGD_2$ erfolgte am pharmakologischen Modell des isolierten perfundierten Kaninchenohres nach ISAKSON et al., modifiziert nach JUAN und SAMETZ (Heinz Juan und Wolfgang Sametz, Naunyn-Schmiedeberg's Arch. Pharmacol. 314, 183-190 (1980)), gemäß der folgenden Versuchsanordnung:

| | |
|---|---|
| Perfusion des Kaninchenohres mit Tyrodelösung 3 ml/min | 20 min |
| $^{14}$C-Arachidonsäure 56 Becquerel in Tyrode gelöst 3 ml/min (recycling) | 60 min |
| Perfusion mit Tyrodelösung + BSA (Rinder-Serum-Albumin) (0,5 mg/ml) 3 ml/min | 30 min |
| Perfusion mit Extrakt 0,5 ml + 2,5 ml Tyrodelösung + BSA/min (Total-volumen 3 ml/min) | bis Ver-suchsende |

Nach 30 min einmalige Stimulierung mit Ca-Ionophore (10 µg Ca$^{++}$-Ionophore A23187).

Tyrodelösung:

| | | |
|---|---|---|
| 8,0 g NaCl | Na$^+$ | 149,2 mmol |
| 0,2 g KCl | K$^+$ | 2,7 mmol |
| 0,26 g CaCl$_2$.2H$_2$O | Ca$^{2+}$ | 1,8 mmol |
| 0,21 g MgCl$_2$.5H$_2$O | Mg | 2,1 mmol |
| 1,0 g NaHCO$_3$ | HCO$_3$– | 11,9 mmol |
| 0,06 g NaH$_2$PO$_4$.2H$_2$O | H$_2$PO$_4$– | 0,4 mmol |
| | Cl– | 145,3 mmol |
| 1,1 g Glucose | Glucose | 5,5 mmol |
| ad 1000 ml H$_2$O dest. | | |

Das Perfusat wird 20 min vor der Ca-Stimulierung über einen Zeitraum von 20 min (Basalfreisetzung) und nach Ca-Stimulierung über einen Zeitraum von 20 min gesammelt (stimulierte Freisetzung). Anschließend werden die Prostaglandine den Perfusaten durch Ausschütteln mit Äthylacetat entzogen, dünnschichtchromatographisch aufgetrennt und die Radioaktivität der einzelnen DC-Zonen (die DC - Laufbahn wird in jeweils 0,4 cm breiten Zonen abgeschabt) mittels eines Szintillationszählers gemessen (vgl. JUAN und SAMETZ).

Das Versuchsschema ist für das Kontrollohr analog, nur wird anstelle der Extraktperfusion Tyrodelösung + BSA perfundiert.

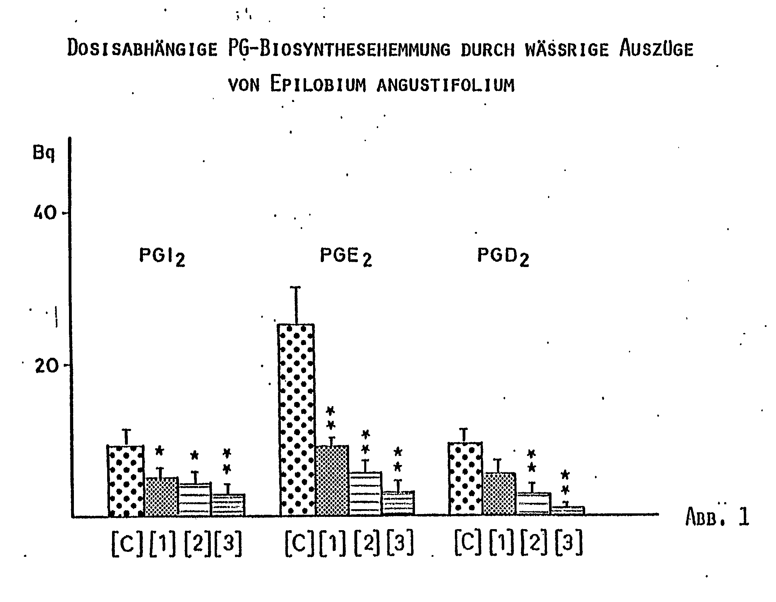

Die Ergebnisse über den Nachweis der dosisabhängigen Beeinflussung wässriger Auszüge von Epilobium angustifolium auf die Biosynthese bzw. Freisetzung der Prostaglandine PGE$_2$, PGI$_2$ und PGD$_2$ sind in **Abbildung** 1 veranschaulicht.

In Abb. 1 bedeuten:

[c] = Kontrolle

[1] = 0,19 ml Extrakt (1,25 mg Drogen-gewicht/ml Perfusionslösung aus 0,19 ml Extrakt und 2,81 ml Tyrodelösung)

[2] = 0,38 ml Extrakt (2,5 mg Drogen-gewicht/ml Perfusionslösung aus 0,38 ml Extrakt und 2,62 ml Tyrodelösung)

[3] = 0,75 ml Extrakt (5 mg Drogengewicht/ml Perfusionslösung aus 0,75 ml Extrakt und 2,81 ml Tyrodelösung)

± SEM (n = 4)

* = p < 0,05

** = p < 0,01

Bq = Becquerel

Der Nachweis der systemischen, entzündungshemmenden Wirkung wässriger Auszüge von Epilobium angustifolium wurde am pharmakologischen Modell des Carrageenin-induzierten Rattenpfotenödems mittels der Quecksilberverdrängungsmethode erbracht:

Weibliche Sprague Dawley Ratten 170 - 200 g wurden über einen Zeitraum von 20 Stunden ohne feste Nahrung belassen. Nach Anaesthesie mit Phenobarbiton (50 mg/kg, i.p.) wurde den Versuchstieren der Extrakt (1 ml/100 g Körpergewicht) mittels Magensonde verabreicht (1 ml entspricht 33 mg Drogengewicht). Nach zwei Stunden wurde in die Plantarregion der rechten hinteren Pfote Carrageenin (0,1 ml, 2 %) und in die Plantarregion der linken hinteren Pfote Natriumchlorid (0,1 ml, 0,9 %) injiziert, und anschließend wurden die Volumina der Pfoten mittels der Quecksilberverdrängungsmethode bestimmt. Das Volumen der mit Natriumchlorid behandelten Pfote wurde mit 0 % angenommen.

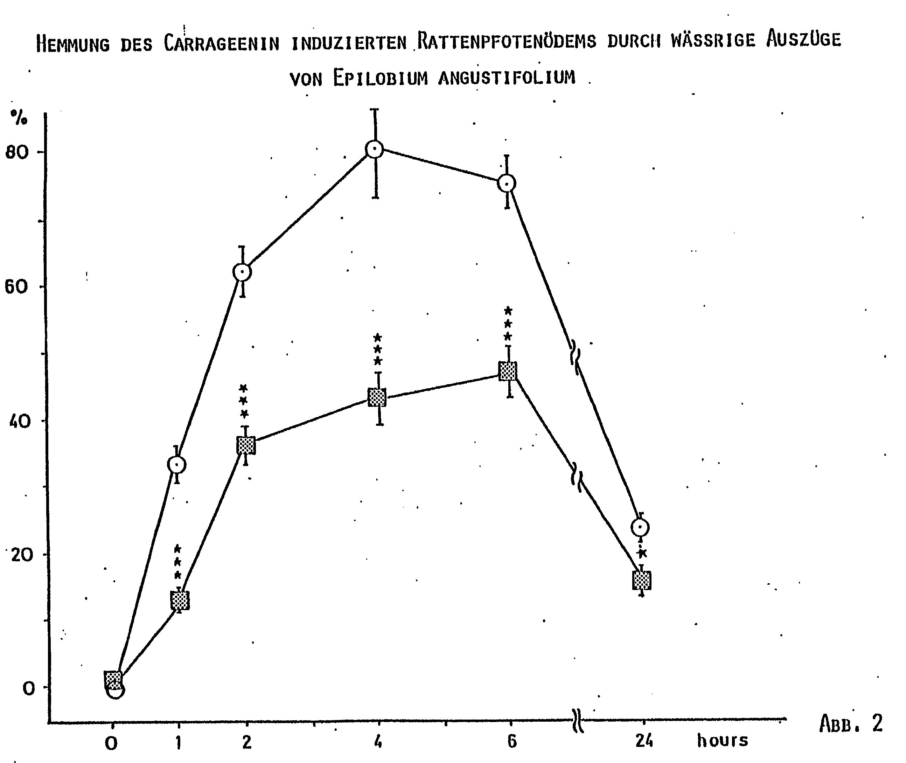

Der Nachweis über die entzündungshemmende Wirkung wässriger Auszüge von Epilobium angustifolium am Carrageenin-induzierten Rattenpfotenödem geht aus **Abbildung** 2 hervor.

In Abb. 2 bedeuten:

$\bigcirc$ = Kontrollgruppe

▨ = 1 ml Extrakt/100 g (1 ml entspricht 33 mg Drogengewich

± SEM (n = 12-15) *p = <0,05 ***p = < 0,001

Die Abbildungen 1 und 2 beziehen sich auf den Nachweis der Prostaglandinfreisetzung und des Rattenpfotenödems nach den vorstehend aufgeführten Modellen unter der hemmenden Wirkung wässriger Auszüge gemäß Beispiel 1. Wässrige Auszüge von Epilobium angustifolium nach Vorextraktion mit organischen Lösungsmitteln (Aceton, Chloroform, Dichlormethan, Äthylacetat, Äthylmethylketon) (vgl. Beispiel 2) zeigen die gleichen Ergebnisse, d.h. es tritt kein Wirkungsverlust ein.

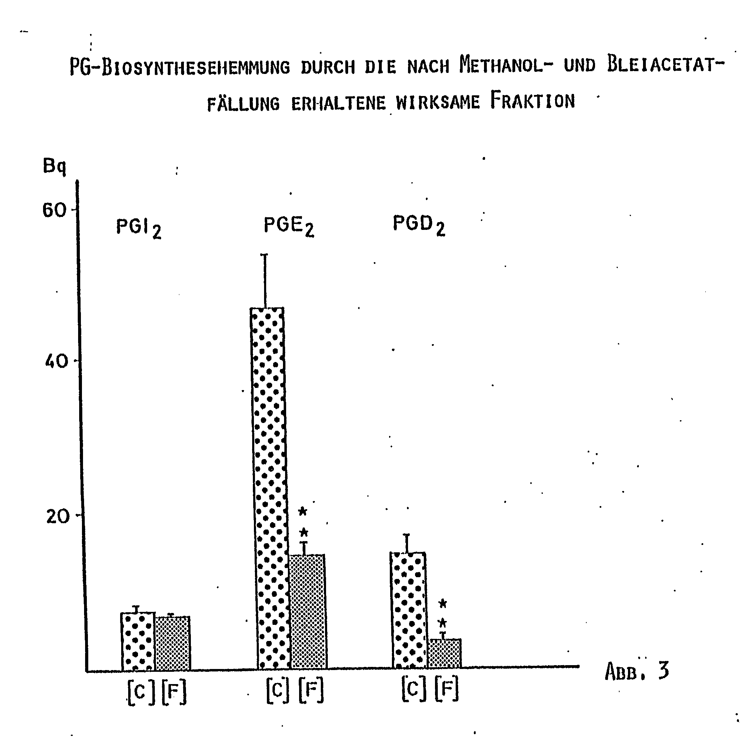

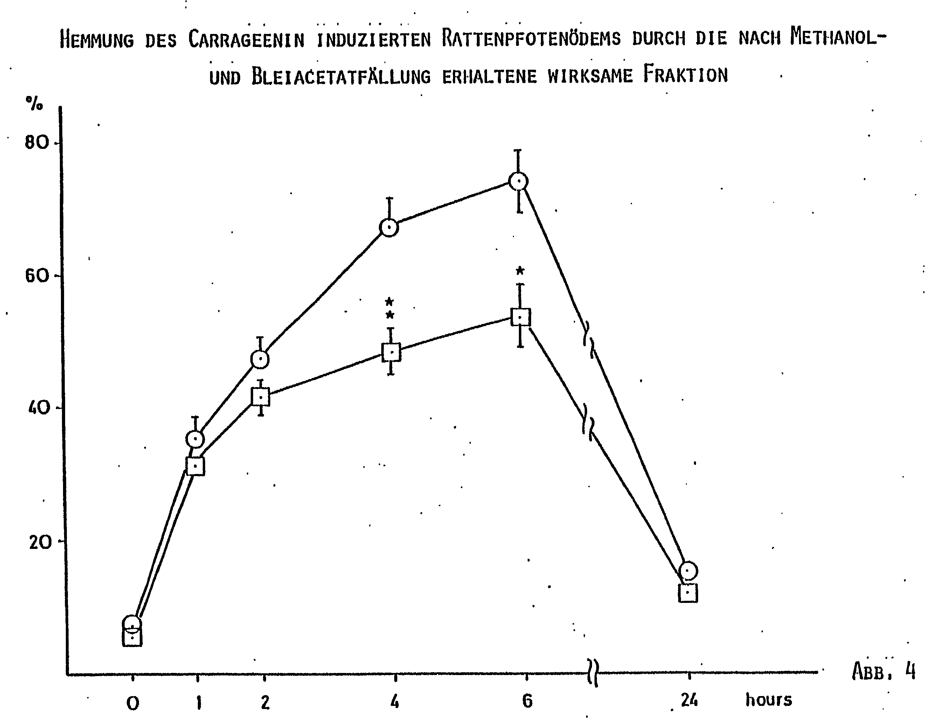

Die **Abbildungen** 3 und 4 veranschaulichen den Einfluß wässriger Auszüge, die zusätzlich gemäß Beispiel 3 behandelt worden sind, auf die PG-Freisetzung und das Carrageenininduzierte Rattenpfotenödem.

In Abb. 3 bedeuten:

[C] = Kontrolle
[F] = 0,5 ml Extrakt (3,3 mg Drogengewicht/ml Perfusionslösung aus 0,5 ml Extrakt und 2,5 ml Tyrodelösung)
± SEM (n = 4) **p = < 0,01
In Abb. 4 bedeuten:

$\bigcirc$ = Kontrollgruppe

□ = 1 ml Extrakt/100 g (1 ml entspricht 33 mg Drogengewich

± SEM (n - 12-15) **p = < 0,01    *p = < 0,05

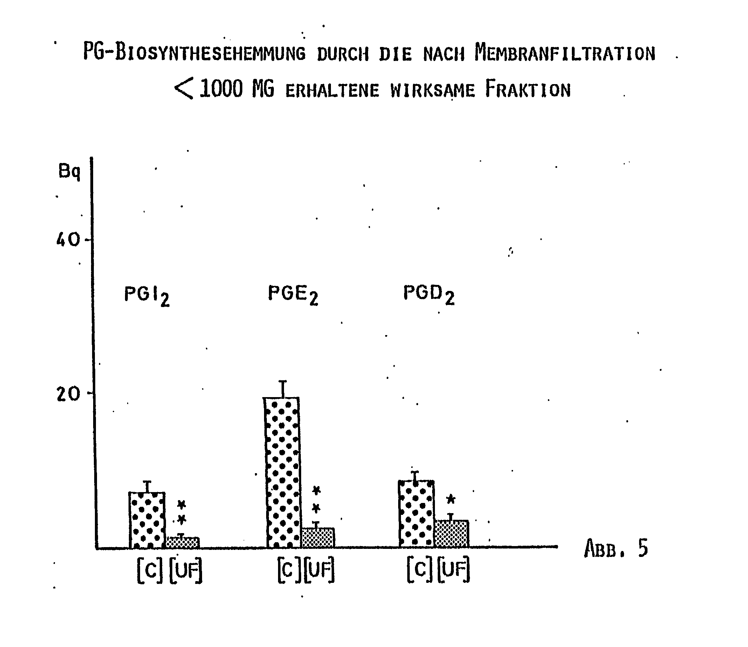

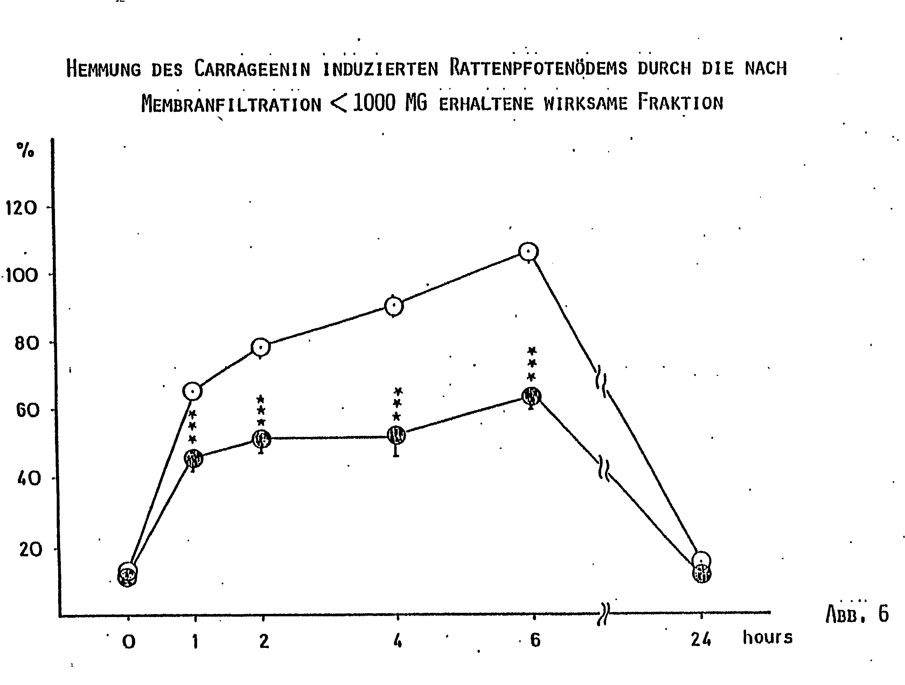

Der Nachweis über die Hemmung der PG-Freisetzung und des Carrageein-induzierten Rattenpfotenödems des gemäß Beispiel 4 hergestellten Extraktes ist in den **Abbildungen** 5 und 6 dargestellt.

In Abb. 5 bedeuten:

[C] = Kontrolle
[UF] = 0,5 ml Ultrafiltrat < 1000 MG (3,3 mg Drogengewicht/ml Perfusionslösung aus 0,5 ml Ultrafiltrat und 2,5 ml Tyrodelösung)
± SEM (n = 4) * = p < 0,05 ** = p < 0,01
In Abb. 6 bedeuten:

$\bigcirc$ = Kontrolle

◉ = 1 ml Ultrafiltrat < 1000 MG/100 g (1 ml entspricht

33 mg Drogengewicht)

± SEM (n = 12-15) *** = p < 0,001

Das Wirkprinzip der Extrakte ist hydrophil und hat ein Molekulargewicht unter 1000. Es handelt sich wahrscheinlich vor allem um phenolische Verbindungen. Es ist in den Blättern lokalisiert. Die Extraktgehalte wässriger Auszüge betragen von Herba und Folium Epilobium angustifolium 30 %, Folium Epilobium angustifolim nach Untrafiltration < 1000 19 %.

**Patentansprüche**

1. Wässrige Auszüge von Epilobium angustifolium L. zur Behandlung entzündlicher Zustände.

2. Wässrige Auszüge von Epilobium angustifolium L. zur Hemmung der Metabolisierung der Arachidonsäure, insbesondere zur Hemmung der Prostaglandin-Synthese und/oder -Freisetzung bei der Behandlung von Krankheiten, die – ganz oder teilweise – auf der Freisetzung von Metaboliten der Arachidonsäure beruhen oder von dieser beeinflußt werden.

3. Verwendung von wässrigen Auszügen von Epilobium angustifolium L. zur Herstellung eines Arzneimittels zur Hemmung der Metabolisierung der Arachidonsäure, insbesondere zur Hemmung der Prostaglandin-Synthese und/oder -Freisetzung bei der Behandlung von Krankheiten, die – ganz oder teilweise – auf der Freisetzung von Metaboliten der Arachidonsäure beruhen oder beeinflußt werden.

4. Verwendung von wässrigen Auszügen von Epilobium angustifolium L. zur Herstellung eines Arzneimittels zur Behandlung entzündlicher Zustände.

**Claims**

1. Aqueous extracts of Epilobium angustifolium L. for treatment of inflammatory conditions.

2. Aqueous extracts of Epilobium angustifolium L. for inhibiting the metabolising of arachidonic acid, especially for inhibiting the synthesis and/or liberation of prostaglandin in the treatment of illnesses, which – completely or partly – are caused by the liberation of metabolites of arachidonic acid or are influenced by these.

3. Use of aqueous extracts of Epilobium angustifolium L. for production of a medicine for inhibiting the metabolising of arachidonic acid, especially for inhibiting the synthesis and/or liberation of prostaglandin in the treatment of illnesses, which – completely or partly – are caused by the liberation of metabolites of arachidonic acid or are influenced by these.

4. Use of aqueous extracts of Epilobium angustifolium L. for production of a medicine for treatment of inflammatory conditions.

**Revendications**

1. Extraits aqueux d'Epilobium angustifolium L. pour le traitement d'états inflammatoires.

2. Extraits aqueux d'Epilobium angustifolium L. pour inhiber la métabolisation de l'acide arachidonique, en particulier pour inhiber la synthèse et/ou la libération des prostaglandines lors du traitement de maladies qui, en totalité ou en partie, se fondent sur la libération de métabolites de l'acide arachidonique ou sont influencées par elle.

3. Utilisation d'extraits aqueux d'Epilobium angustifolium L. pour la préparation d'un médicament destiné à inhiber la métabolisation de l'acide arachidonique, en particulier pour inhiber la synthèse et/ou la libération des prostaglandines lors du traitement de maladies qui, en totalité ou en partie, se fondent sur la libération de métabolites de l'acide arachidonique ou sont influencées par elle.

4. Utilisation d'extraits aqueux d'Epilobium angustifolium L. pour la préparation d'un médicament destiné au traitement d'états inflammatoires.

DOSISABHÄNGIGE PG-BIOSYNTHESEHEMMUNG DURCH WÄSSRIGE AUSZÜGE
VON EPILOBIUM ANGUSTIFOLIUM

ABB. 1

HEMMUNG DES CARRAGEENIN INDUZIERTEN RATTENPFOTENÖDEMS DURCH WÄSSRIGE AUSZÜGE VON EPILOBIUM ANGUSTIFOLIUM

ABB. 2

PG-BIOSYNTHESEHEMMUNG DURCH DIE NACH METHANOL- UND BLEIACETAT-
FÄLLUNG ERHALTENE WIRKSAME FRAKTION

ABB. 3

EP 0 234 019 B1

HEMMUNG DES CARRAGEENIN INDUZIERTEN RATTENPFOTENÖDEMS DURCH DIE NACH METHANOL- UND BLEIACETATFÄLLUNG ERHALTENE WIRKSAME FRAKTION

ABB. 4

EP 0 234 019 B1

PG-BIOSYNTHESEHEMMUNG DURCH DIE NACH MEMBRANFILTRATION $< 1000$ MG ERHALTENE WIRKSAME FRAKTION

ABB. 5

Abb. 6